# EUROPEAN PATENT SPECIFICATION

(11) **EP 1 397 660 B1**
(45) Date of publication and mention of the grant of the patent: **15.05.2013**
(21) Application number: 02733196.6
(22) Date of filing: 20.05.2002
(51) Int. Cl.: A61B 5/00

(54) **A FLOATABLE IN VIVO SENSING DEVICE**
SCHWIMMFÄHIGE IN-VIVO-SENSORVORRICHTUNG
APPAREIL D'ANALYSE IN VIVO CAPABLE DE FLOTTER

(30) Priority: 20.05.2001 IL 14325901; 14.06.2001 US 297761 P
(43) Date of publication of application: 17.03.2004
(73) Proprietor: Given Imaging Ltd., 20692 Yoqneam (IL)
(72) Inventor: LEWKOWICZ, Shlomo, 36031 Tivon (IL); GAT, Daniel, Nesher, 36863 Haifa (IL); GLUKHOVSKY, Arkady, 36790 Nesher (IL); KHAIT, Semion, 14350 Tiberias (IL)
(74) Representative: Graf, Werner
(86) International application number: PCT/IL2002/000392
(87) International publication number: WO 2002/095351

(56) References cited:
- WO-A-00/33738
- WO-A1-00/22975
- WO-A1-00/54701
- WO-A1-01/10291
- JP-A- 2000 342 527
- US-A- 3 144 017
- US-A- 5 279 607
- US-A- 5 604 531
- US-A- 6 099 482
- US-A- 6 149 581
- US-B1- 6 240 312
- IDDAN G ET AL: "WIRELESS CAPSULE ENDOSCOPY THE DISCOMFORT OF INTERNAL GASTROINTESTINAL EXAMINATION MAY SOON BE A THING OF THA PAST" NATURE, NATURE PUBLISHING GROUP, LONDON, UK, vol. 405, no. 6785, 1 May 2000 (2000-05-01), page 417, XP000915437 ISSN: 0028-0836

## Description

### FIELD OF THE INVENTION

The present invention relates to the field of in vivo sensing. Specifically, the present invention relates to a floatable in vivo sensing device that can be carried by liquid.

### BACKGROUND OF THE INVENTION

In vivo sensors are non invasive tools used in diagnosis of diverse body systems. For example, ingestible devices may be used for sensing in vivo conditions in the gastrointestinal (GI) tract, such as, temperature, pH or pressure. Ingestible imaging devices can be used for imaging the gastrointestinal tract. For example, a capsule comprising a sensor, such as an image sensor, may be ingested and moved passively through the small intestine by peristalsis while imaging or otherwise sensing the small intestine. Such a capsule is disclosed in IDDAN G ET AL: "WIRELESS CAPSULE ENDOSCOPY THE DISCOMFORT OF INTERNAL GASTROINTESTINAL EXAMINATION MAY SOON BE A THING OF THA PAST" NATURE, NATURE PUBLISHING GROUP, LONDON, UK, vol. 405, no. 6785, 1 May 2000 (2000-05-01), page 417, However, passive movement of objects through larger body lumens, such as the large intestine, may be slower and unpredictable. The large intestine, or colon, whose main function is to remove much of the water from the stool and to store the stool, begins with the cecum, a small saclike evagination, then continues with the ascending colon, from the appendix in right groin up to a flexure at the liver, transverse colon, liver to spleen, descending colon, spleen to left groin, then sigmoid (S-shaped) colon back to midline and anus. The colon has three longitudinal muscle bands whose actions assist movement through the colon.

It is sometimes advantageous to move objects through the colon independently of the natural action of the colon muscles. For example, delivery of a medicament to a specific location in the colon may be time dependant and cannot rely on the natural movement in the colon. Also a device for imaging the colon might benefit from being actively moved through the colon so as to efficiently view the colon.

Current methods of moving objects, especially imaging devices, through the colon involve the use of endoscopes, typically colonoscopes, which are expensive and inconvenient for patient use, and do not always enable to reach distal parts of the colon, such as the cecum and the right colon.

### SUMMARY OF THE INVENTION

The present invention provides an essentially floatable sensing device that can be carried by liquid. The sensing device according to the invention is useful in sensing, such as by imaging, lumens containing or capable of containing a bulk of liquid. One example of such a lumen is the large intestine. The essentially floatable sensing device of the invention is carried by the liquid and can thus be moved through the lumen together with the bulk of liquid.

According to the invention the in vivo sensing device has a specific gravity (SG) of about 1.0 or a volume to weight ratio that enables it essentially to float.

The in vivo sensing g device comprises a sensor system and a buoyant body. The buoyant body, which can optionally house one or more elements of the sensor system, may have a specific gravity of about 1.0, which is just about floating, or a volume to weight ratio that enables it essentially to float in a body lumen liquid. The buoyant body may be a buoy attached to a sensor system, which keeps the sensor system essentially floating in a body lumen liquid.

The sensor system may comprise any sensor suitable for sensing in vivo environment parameters, such as pH, temperature, conductivity, shear, pressure and so on. The sensor system can include all the elements necessary for in vivo sensing, as known in the art. In one embodiment the sensor is an image sensor.

The sensing device according to the present invention can be used for sensing the large intestine. Typically, the method involves moving an in vivo sensor through the body lumen. high osmotie pressure compositions, such as contrast agents, are utilized to assist in the movement of objects through the colon.

### BRIEF DESCRIPTION OF THE DRAWINGS

The present invention will be understood and appreciated more fully from the following detailed description talon in conjunction with the drawings in which:
Figure 1 is a schematic illustration of a floatable sensing device in accordance with an embodiment of the invention;
Figure 2A is a schematic illustration of a floatable sensing device in accordance with another embodiment of the invention;
Figure 2B is a schematic illustration of a floatable sensing device with a packaged buoy in accordance with an embodiment of the invention; and
Figures 3A and 3B are schematic illustrations of a floatable sensing device in accordance with yet other embodiments of the invention.

### DETAILED DESCRIPTION OF THE INVENTION

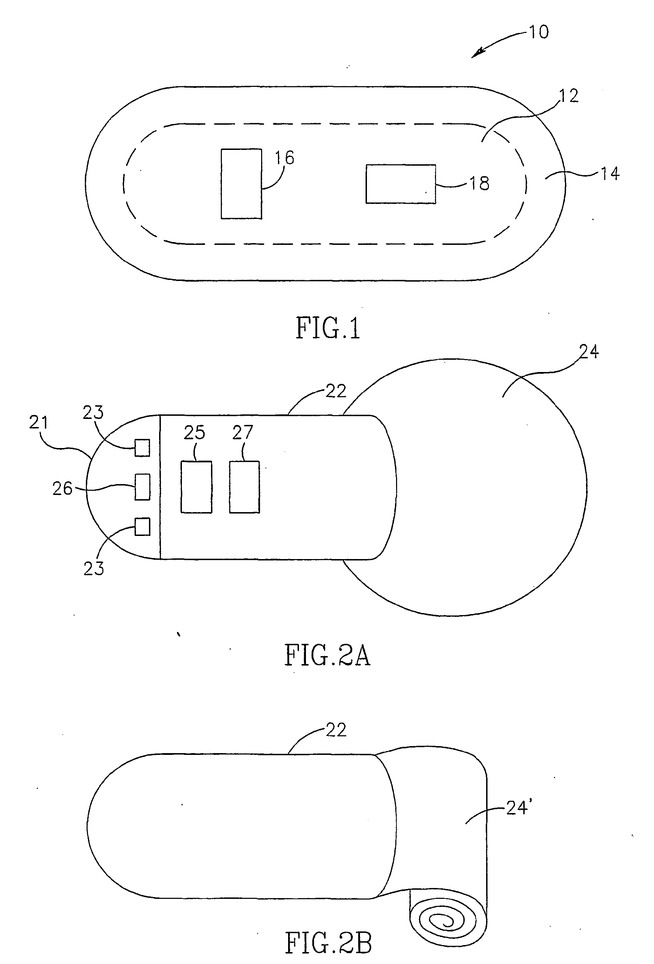

A sensing device, according to an embodiment of the invention, is schematically illustrated in Fig 1. The sensing device 10 is an autonomous capsule shaped device and can thus be easily moved through the small intestine. However, it should be appreciated that an autonomous sensing device can have any shape or design suitable for being inserted and moved through body lumens, such as the gastrointestinal tract, the reproductive system, blood vessels, etc.

Device 10 includes a sensor system 12 and a buoyant body 14. In one embodiment, the buoyant boy 14 is essentially a part of the sensor system 12. The sensor system 12 may comprise a pH sensing system as known in the art or other known in vivo sensing systems such as an in vivo temperature measuring system, a pressure sensor, a shear sensor, a sensor of electrical conductivity and other known in vivo sensors. In other embodiments a combination of sensors may be used. In the invention the sensor system 12 comprises an imaging device comprising, for example, at least one image sensor (such as image sensor 16), one or more illumination source (not shown) and one or more transmitter for transmitting image signals to an external receiving system (not shown). In vivo imaging systems that can be utilized with the present invention are described, for example, with reference to Fig. 2A below, or in WO 01/65995 or in US Patent Number 5,604,531. In one embodiment the sensor system 12 further includes a temperature measuring system. A temperature measuring system according to an embodiment of the invention may comprise an image sensor 16 having an image sensing module in communication with an integrating unit (not shown) for detecting the dark current of the image sensor image sensing module and for calculating the temperature of the image sensor. The integrating unit further calculates the temperature of the environment or the temperature of the environment may be calculated, based on data from the integrating unit, by a separate unit that is in communication with the integrating unit. A temperature measuring system that may be utilized according to an embodiment of the invention is described, for example, in WO 01/10291.

Typically, the sensor system 12 is powered by a battery 18, however other embodiments are possible, for example, the sensor system may be power induced by an external electromagnetic field.

The buoyant body 14 lends a volume to weight ratio, or a specific gravity to the device 10 that enables it to float in a liquid filled lumen.

SG - specific gravity - is weight [gr.] / weight of water in the amount displaced by the device 10 (when reaching maximal volume) or for sealed objects, SG is weight [gr] / volume [cubic cm]. Typically, buoyant body 14 can be filled with a substance lighter than the body lumen liquid, such as gaseous CO₂, O₂ or air.

In another embodiment of the invention schematically illustrated in Fig.2A, the sensor system is an image sensor system 22 and the buoyant body is an inflatable buoy 24. The buoy 24 may be packaged such that it is not buoyant while in packaging. However, release of the buoy from its packaging lends buoyancy to the sensor system. The buoy may be released from its packaging at a desired location or point in time, such that the sensor system 22 may acquire buoyancy according to specific requirements. For example, the floatable sensing device according to an embodiment of the invention may be ingested and moved by peristalsis through the small intestine while its buoy is packaged. When the device enters the large intestine the buoy is released from its packaging and the device can then float in the bulk of liquid in the large intestine and be carried by the bulk of liquid to all areas of the large intestine. Thus, the device is moved through the large intestine, effectively sensing the lumen. The mechanisms by which the buoy is released from its packaging can be externally controlled or automatically controlled as will be further described below.

The image sensor system 22 includes an image sensor 26, for example a charge coupled device (CCD) or a complementary metal oxide semiconductor (CMOS) image sensor, illumination sources 23, an optical system which includes an optical window 21 through which the body lumen is illuminated and imaged and lenses and/or mirrors (not shown) for collimating light, a transmitter 25 for transmitting image signals to an external receiving system (not shown) and a battery 27 for supplying power to the elements of the image sensor system 22.

The inflatable buoy 24 is a typically elastic compartment containing air, which is attached to the image sensor system 22. The inflatable buoy 24 will lend buoyancy to the image sensor system 22 such that it can essentially float in the liquid filling a body lumen and be thus moved through the body lumen, obtaining images of essentially all parts of the body lumen.

In another embodiment of the invention, schematically illustrated in Fig. 2B an image sensor system 22, as above, comprises an inflatable buoy 24'. The inflatable buoy 24' is rolled and packaged to a small size. In its packaged form inflatable buoy 24' typically does not lend buoyancy to the image sensor system 22. The inflatable buoy 24' may be released from its packaging by a manually or automatically controlled mechanism, at a desired location in vivo. For example, the inflatable buoy 24' may contain gas releasing granules such as crystalline sodium bicarbonate, E-Z GasII effervescent granules by EZEM of NY, USA or similar oxygen releasing granules. Typically, these granules release gas (such as CO₂ or oxygen) upon contacting liquid. In an embodiment of the invention inflatable buoy 24'contains two compartments (not shown), one compartment containing gas releasing granules (for example 100 mg of granules) and the other containing a drop of liquid (for example 0.1 cc of water or saline). The compartments are kept separate while the inflatable buoy 24' is in its packaged form. Once the packaging is opened, the two compartments are merged and the drop of liquid contacts the gas releasing granules. Gas is released into the now unpacked buoy inflating the buoy and enhancing buoyancy.

The packaging can be achieved by a manually controlled mechanism, such as a bimorph material mechanism that could change configuration in accordance with controllable conditions, such as a temperature or electric voltage gradient, as known in the art. Alternatively, the packaging mechanism can be time dependent or dependant on in vivo conditions such as pH or enzymatic activity. For example, packaging can be effected by a hydrocarbon such as a gelatin capsule which encases the image sensor system 22 and the inflatable buoy 24' and keeps the inflatable buoy 24' in a packaged form. According to one embodiment, the gelatin capsule slowly dissolves in the liquid present in the stomach, thereby releasing the inflatable buoy. The gelatin capsule, such as gelatin capsules provided by Capsugel USA, can be made to dissolve at a specific location along the GI tract, as known in the field of sustained release mechanisms. Thus, a device comprising image sensor system 22 and inflatable buoy 24' can be encased in a hydrocarbon capsule while moving through certain parts of a patient's GI tract and free of the encapsulation in other parts of the GI tract. While free of the encapsulation, inflatable buoy 24' will be inflated and the device will be able to essentially float in the GI tract liquid. In another embodiment the packaging can be effected by degradable sutures known in the art, such that the packaging of the inflatable buoy 24' is released when the suture is degraded.

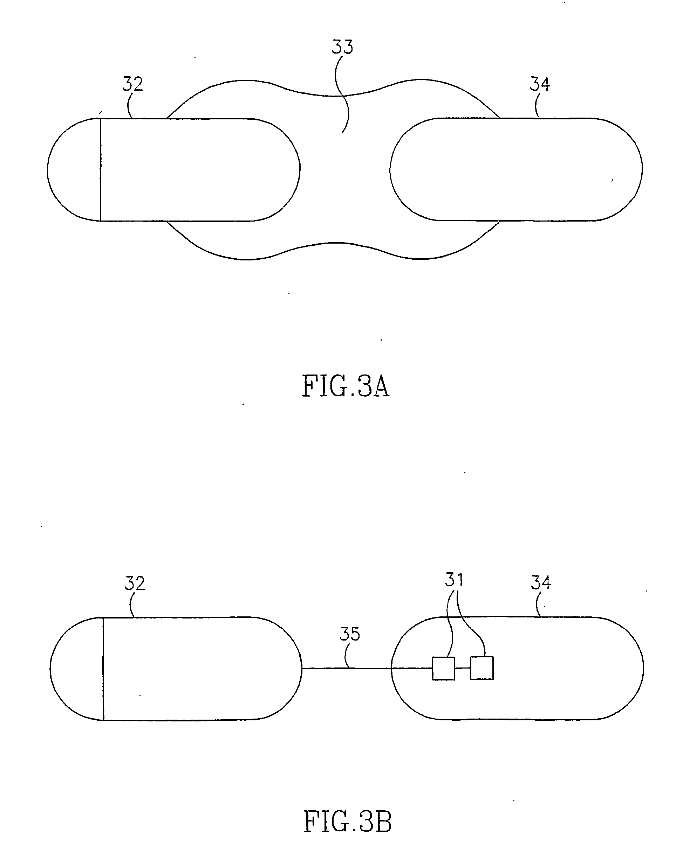

Another embodiment of the invention is schematically illustrated in Figs. 3A and 3B, in which a sensor system 32, such as an imaging sensor system or other sensing systems, for example, those described above, is attached to a buoy 34, which is typically a light vessel made of, for example, plastic such as isoplast, filled with a substance lighter than the body lumen liquid. In certain embodiments of the invention the buoy 34 can contain accessory components or materials, such as additional batteries 31.

The sensor system 32 and the buoy 34 can be attached by a flexible sleeve 33 (Fig. 3A), which, if filled with a substance lighter than the body lumen liquid, can also assist in enhancing the buoyancy of the sensor system 32. Alternatively, the sensor system 32 and the buoy 34 can be attached by a wire 35 (Fig. 3B). The wire 35 can couple sensor system 32 and the buoy 34, for example, if electrical coupling is needed for utilizing the additional batteries 31 by the sensor system 32.

According to a method for sensing body lumens, such as the stomach or large intestine, a floatable sensing device, such as the devices described above, is inserted in vivo. The sensing device may include a sensor system and a buoyant body. The buoyant body may be inflated prior to being inserted in vivo or may be inflated at a specific location or point in time while in a patient's body, as described above. Preferably, the sensor system and buoyant body are placed in a body lumen containing a bulk of liquid. More preferably there is a flow of liquids through the body lumen, upon which the sensor system is carried through the body lumen. Thus, this method can be used, in one embodiment, for imaging or otherwise sensing a patient's large intestines. The patient's large intestine is initially cleared of its contents, for example by inducing bowel movement by administering a laxative or an enema. Further, the patient's large intestine is filled with a liquid, for example by drinking high osmolarity liquids, which retain liquids within the large intestine for longer periods. Typically, a liquid loaded intestine and/or additional laxatives, administered while the sensor system is in the large intestine, will induce bowel movement and cause a flow of the bulk of liquids in the large intestine. The induced flow will enhance movement of the sensor system through the large intestine, thereby facilitating sensing of most areas of the lumen.

An imaging device for imaging the GI tract can be moved through the large intestine by utilizing, for example, a high osmotic pressure composition that is essentially not absorbed by the intestine. Alternatively, an object other than an imaging device, for example a device for sustained release of medicaments to the colon, can be moved through the large intestine by utilizing a high osmotic pressure composition that is essentially not absorbed by the intestine. Objects moved through the large intestine utilizing a high osmotic pressure composition may or may not be floatable.

A wireless in vivo imaging device such as the sensor systems described above or a device including a compartment for collecting or distributing substances from or to the GI tract, such as the device described in WO00/22975, can be moved through the large intestine such that it can be used for diagnostic and/or for therapeutic purposes.

An in vivo imaging device can be introduced into the GI tract by utilizing a colonoscope. The in vivo imaging device can be ingested by a patient and traverses the small intestine pushed along by natural peristalsis. When the device reaches the cecum it may remain in the cecum for typically long periods of time. A high osmotic pressure composition that is essentially not absorbed by the intestine, such as a contrast agent, may be administered to the patient. The high osmotic pressure composition typically progresses through the small intestine arriving at the cecum. The composition usually progresses in the intestine faster than the device, pushing in its progression the device through the different parts of the colon. An example of a high osmotic pressure composition that may be used according to an of the invention is barium sulfate or gastrografin. The osmotic pressure of gastrografin at 37°C is 55.1 Atm and its osmolality is 2.15 (osm/kg H₂O).

A wireless ingestible imaging capsule as described above was administered under Helsinki Committee guidelines to normal healthy volunteers in the standard fashion. The volunteers underwent standard colonoscopic preparation consisting of a 24-hour liquid diet and 4 ounces of Fleet phosphasoda the evening before and the morning of the procedure. The volunteers were encouraged to drink a large volume of fluid before and after ingestion of the capsule. The passage of the capsule was monitored on-line. The volunteers were given 8 ounces of gastrografin diluted as for regular body CT examination [5%], every 15 minutes starting 2.5 hours after ingestion of the capsule, up to a total of 2 liters. The volunteers were allowed to eat a normal meal 4 hours after ingestion of the capsule.

**Results:** In one case, the gastric emptying time was 42 minutes, and the capsule reached the cecum after 4 hours and 16 minutes. The capsule was excreted from the colon 22 hours after ingestion. Images of various parts of the colon were acquired for more than 3 hours. In the second case, gastric emptying occurred after 8 minutes, and the capsule reached the cecum after 8 hours. Images of all parts of the colon were acquired for 2.5 hours. The capsule was excreted after 10.5 hours, still working.

**Conclusion:** The first successful passage through the right colon in the first case and the entire colon in the second case of a functioning imaging capsule using a novel technique is described. The use of gastrografin was based on clinical observation in GI radiological procedures, such as CT. This agent may have an important role in the movement of the imaging capsule through the colon.

Gastrografin contains a mixture of sodium amidotrizoate and meglumine amidotrizoate in a proportion of 10:66 (amidotrizoic acid or diatrizoic acid: 3,5-bis-acetamido-2,4,6-triiodobenzoic acid). 1 mL gastrografin contains sodium amidotrizoate 100,00 mg and meglumine amidotrizoate 660,00 mg (sodium diatrizoate and meglumine diatrizoate) in aqueous solution plus flavorings and a wetting agent. The contrast-giving substances in gastrografin are salts of amidotrizoic acid in which the X-ray absorbing iodine is present in stable chemical bond. Following oral administration only about 3% of the amidotrizoic acid is absorbed by the stomach and intestines. This portion is eliminated mainly via the kidneys.

Gastrografin can be used either orally or as an enema. Gastrografin typically travels through the colon faster than the capsule and is a clear fluid, allowing a clear view of the colon. Another advantage of gastrografin is its opacity to x-ray, allowing direct view in fluoroscopy and enabling monitoring of the passage of the capsule through the GI tract.

It will be appreciated by persons skilled in the art that the present invention is not limited to what has been particularly shown and described hereinabove. Rather the scope of the present invention is defied only by the claims which follow:

## Claims

1. An essentially floatable in vivo imaging device (10) for moving together with a bulk of liquid through a large intestine and for imaging the large intestine, wherein the imaging device (10) is capsule shaped and ingestible,
wherein the imaging device (10) includes
- a sensor system (12);
- and a buoyant body (14);
and wherein the sensor system (12) comprises:
- an illumination source; and
- an image sensor (16) ; and
- a transmitter (25) for transmitting image signals to an external receiving system;
and wherein the buoyant body (14) lends a volume to weight ratio to the imaging device (10) in an amount such that the imaging device (10) has a specific gravity relative to water of about 1.

2. The in vivo imaging device according to claim 1 further comprising at least one in vivo sensor.

3. The in vivo imaging device according to claim 1 or 2, wherein the buoyant body (14) houses a sensor system (12).

4. The in vivo imaging device according to claim 2, wherein the in vivo sensor is selected from the group consisting of: a pH meter, a temperature sensor, a pressure sensor, a shear sensor, a sensor of electrical conductivity or a combination thereof.

5. Use of the device according to one of the preceding claims in sensing the large intestine after insertion of said device into a body lumen.

## Patentansprüche

1. Eine im Wesentlichen schwimmfähige In-vivo-Bilderzeugungsvorrichtung (10) zum Bewegen durch einen Dickdarm, gemeinsam mit einer Menge Flüssigkeit, und zum Abbilden des Dickdarms, wobei die Bilderzeugungsvorrichtung (10) kapselförmig und einnehmbare ausgestaltet ist,
wobei die Bilderzeugungsvorrichtung (10) umfasst
- ein Sensorsystem (12);
- und einen Auftriebskörper (14);
und wobei das Sensorsystem (12) umfasst:
- eine Beleuchtungsquelle; und
- einen Bildsensor (16); und
- einen Sender (25) zum Übertragen von Bildsignalen zu einem externen Empfängersystem;
und wobei der Schwimmkörper (14) der Bilderzeugungsvorrichtung (10) ein derartiges Volumen-Gewichts-Verhältnis verleiht, dass die Bilderzeugungsvorrichtung (10) relativ zu Wasser ein spezifisches Gewicht von etwa 1 aufweist.

2. In-vivo-Bilderzeugungsvorrichtung gemäss Anspruch 1, ferner umfassend zumindest einen In-vivo-Sensor.

3. In-vivo-Bilderzeugungsvorrichtung gemäss Anspruch 1 oder 2, wobei im Schwimmkörper (14) ein Sensorsystem (12) untergebracht ist.

4. In-vivo-Bilderzeugungsvorrichtung nach Anspruch 2, wobei der In-vivo-Sensor ausgewählt ist aus der Gruppe bestehend aus: ein pH-Messgerät, ein Temperatursensor, ein Drucksensor, ein Scherungssensor, ein Sensor für elektrische Leitfähigkeit oder einer Kombination davon.

5. Verwendung der Vorrichtung nach einem der vorhergehenden Ansprüche zum Erfassen des Dickdarms, nach dem Einführen der genannten Vorrichtung in ein Körperlumen.

## Revendications

1. Dispositif d'imagerie in vivo (10) sensiblement en mesure de flotter destiné à se déplacer en même temps qu'une bulle de liquide à travers un gros intestin et à réaliser une imagerie du gros intestin, où le dispositif d'imagerie (10) est une capsule dimensionnée et pouvant être ingérée,
où le dispositif d'imagerie (10) inclut
- un système de capteur (12) ;
- et un corps flottant (14) ;
et où le système de capteur (12) comprend :
- une source d'illumination ; et
- un capteur d'image (16) ; et
- un dispositif de transmission (25) destiné à transmettre des signaux d'image à un système de réception externe ;
et où le corps flottant (14) confère un rapport de volume sur masse au dispositif d'imagerie (10) dans une mesure telle que le dispositif d'imagerie (10) présente une densité par rapport à l'eau d'environ 1.

2. Dispositif d'imagerie in vivo selon la revendication 1, comprenant en outre au moins un capteur in vivo.

3. Dispositif de d'imagerie in vivo selon la revendication 1 ou 2, dans lequel le corps flottant (14) loge un système de capteur (12).

4. Dispositif d'imagerie in vivo selon la revendication 2, dans lequel le capteur in vivo est sélectionné à partir du groupe constitué : d'un pH-mètre, d'un capteur de température, d'un capteur de pression, d'un capteur de cisaillement, d'un capteur de conductivité électrique ou d'une combinaison de ceux-ci.

5. Utilisation du dispositif selon l'une des revendications précédentes, lors de la détection du gros intestin après insertion dudit dispositif dans une lumière corporelle.
